Europäisches Patentamt

European Patent Office

Office européen des brevets

(19)

(11) **EP 0 493 574 B1**

(12) **FASCICULE DE BREVET EUROPEEN**

(45) Date de publication et mention
de la délivrance du brevet:
**28.02.1996 Bulletin 1996/09**

(21) Numéro de dépôt: **91913613.5**

(22) Date de dépôt: **17.07.1991**

(51) Int Cl.[6]: **C07C 2/76**

(86) Numéro de dépôt international:
**PCT/FR91/00589**

(87) Numéro de publication internationale:
**WO 92/01656 (06.02.1992 Gazette 1992/04)**

(54) **PROCEDE DE CONVERSION DU METHANE EN HYDROCARBURES SUPERIEURS**

VERFAHREN ZUR UMSETZUNG VON METHAN ZU HÖHEREN KOHLENWASSERSTOFFEN

METHOD FOR CONVERTING METHANE INTO HIGHER HYDROCARBONS

(84) Etats contractants désignés:
**AT BE CH DE DK ES GB GR IT LI LU NL SE**

(30) Priorité: **20.07.1990 FR 9009340**

(43) Date de publication de la demande:
**08.07.1992 Bulletin 1992/28**

(73) Titulaire: **GAZ DE FRANCE**
**F-75017 Paris (FR)**

(72) Inventeurs:
• AMARIGLIO, Henri
  **F-54000 Nancy (FR)**
• SAINT JUST, Jacques, Jean
  **F-78230 Le Pecq (FR)**

(74) Mandataire: **Durand, Yves Armand Louis et al**
**F-75008 Paris (FR)**

(56) Documents cités:
EP-A- 0 316 075          GB-A- 2 148 935
GB-A- 2 213 828          US-A- 4 450 310
US-A- 4 634 800

• WO86/07351

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

## Description

La présente invention a pour objet un procédé de conversion du méthane en hydrocarbures supérieurs et plus particulièrement en hydrocarbures $C_2$ à $C_7$.

La conversion du méthane en hydrocarbures supérieurs et hydrogène est défavorisée thermodynamiquement. A ce jour, la voie principale de conversion du méthane est la réaction endothermique de fabrication de gaz de synthèse par réformage à la vapeur. Cette voie a des limites liées à la thermodynamique et à la mise en oeuvre qui ont conduit à la recherche d'alternatives. Dans la recherche de réactions thermodynamiques favorables, une solution a été récemment étudiée, le couplage oxydant, qui consiste à faire réagir catalytiquement le méthane en présence (WO 86/07351, US-A-4 634 800, EP-A-0 316 075) ou en absence (US-A-4 450 310, GB-A-2 148 935, GB-A-2 213 828) d'oxygène pour le convertir en un mélange d'éthane et d'éthylène. Cependant, cette première solution s'est révélée insatisfaisante parce qu'elle n'aboutit pas à un procédé suffisamment sélectif pour la production àes hydrocarbures supérieurs. En effet, en plus de la formation inévitable d'eau, elle conduit à une formation importante de produits secondaires indésirables et notamment à celle d'oxydes de carbone.

La présente invention est basée sur la découverte inattendue que l'on peut obtenir une conversion du méthane sans co-réactif gazeux avec une sélectivité maximale et sans perte importante de matière en contournant les difficultés thermodynamiques et cinétiques par un processus cyclique original associé à un choix de catalyseurs adaptés.

Aussi, la présente invention a pour objet un procédé de conversion du méthane en hydrocarbures supérieurs qui consiste à exposer un catalyseur constitué essentiellement d'un métal appartenant à la série des métaux de transition et d'un support de grande surface spécifique qui est constitué notamment d'oxyde d'un métal réfractaire à un flux de méthane, à une température comprise entre environ 100 et 300°C avec un temps de contact d'au moins une seconde, puis à un flux d'hydrogène à une température comprise entre 100 et 300°C avec un temps de contact d'au moins une seconde.

Selon une caractéristique de l'invention, le catalyseur est constitué d'un métal choisi dans le groupe consistant en Pt, Ru, Pd, Ir, Rh, Re, Co, Ni, Fe, W et leurs mélanges mécaniques et/ou leurs alliages et leurs promoteurs à métaux notamment alcalins ou alcalino-terreux et d'un support de grande surface spécifique qui est constitué d'oxyde d'un métal réfractaire choisi notamment dans le groupe consistant en silice, alumine, silice-alumine, oxydes de zinc, magnésium, chrome, zirconium et terres rares ou de charbon actif.

Selon encore une caractéristique de l'invention, la température est comprise entre environ 130°C et 250°C.

Selon encore une autre caractéristique, le temps de contact est compris entre 12 et 480 secondes.

Selon toujours une caractéristique de l'invention, le procédé consiste de plus à récupérer le flux d'hydrogène en séparant l'hydrogène des produits obtenus.

Selon une particularité de l'invention, un complément d'hydrogène peut être obtenu par réformage catalytique du mélange des produits obtenus.

Selon une autre particularité de l'invention, le procédé consiste en une mise en oeuvre successive et non interrompue des étapes décrites précédemment.

D'autres buts, détails et avantages de la présente invention apparaîtront plus clairement à la lecture de la description détaillée qui va suivre.

Plus particulièrement, le cycle élémentaire du procédé consiste à exposer successivement le catalyseur à un flux de méthane et ensuite à de l'hydrogène. Le procédé consiste en la répétition successive en continu du cycle élémentaire. Ceci peut être réalisé notamment soit en passant sur le catalyseur de façon alternée un flux de méthane et un flux d'hydrogène, soit en faisant circuler le catalyseur successivement dans du méthane puis dans de l'hydrogène.

Dans le premier cas, le catalyseur peut être sous la forme d'un lit fixe, en particulier sous forme de monolithe, mais de préférence il est fluidisé pour assurer un temps de contact identique entre toutes les particules de catalyseur et le méthane ainsi qu'une température uniforme.

Dans le deuxième cas, le catalyseur est sous forme d'un lit fluidisé circulant.

Lorsque la réaction de conversion proprement dite est achevée, un flux gazeux composé d'hydrogène et d'un mélange d'alcanes légers ($C_2$-$C_5$) est recueilli. Une séparation est effectuée entre l'hydrogène, destiné à être recyclé, et les alcanes légers qui sont les produits recherchés.

Il faut noter que bien que le cycle complet de conversion soit globalement producteur d'hydrogène

$$[(CH_4 \rightarrow C_nH_{2n+2} + (n - 1)H_2],$$

il est en pratique consommateur puisque l'hydrogène est produit de façon très dilué, et donc non récupérable, dans le flux de méthane lors de la première étape du procédé qui correspond à l'adsorption et à la condensation du méthane adsorbé. La seconde étape, c'est-à-dire le balayage à l'hydrogène est consommatrice d'hydrogène. La limite supérieure de la consommation d'hydrogène peut être évaluée en considérant que tous les alcanes qui sont alors produits sont adsorbés sous forme d'oléfines. La consommation d'hydrogène est la quantité nécessaire pour transformer les oléfines en alcanes. On aboutit ainsi à environ 70 tonnes d'hydrogène consommées par tonne de produit $C_2$ - $C_5$ soit une quantité

comparable à celles qui sont rencontrées en hydrotraitement de coupes pétrolières. La consommation d'hydrogène joue un rôle important dans la compétitivité des procédés.

Le rendement dépend de la fréquence à laquelle le processus cyclique peut être répété. Les temps de contact les plus brefs possibles sont donc recherchés tels que des temps de contact de 12 à 480 secondes ou moins. Les catalyseurs les plus actifs permettent de réduire le temps de contact nécessaire à l'adsorption, à la conversion du méthane et à la désorption des produits obtenus. Après la désorption, le catalyseur est ainsi régénéré et le processus cyclique prêt à être à nouveau engagé.

Comme on va le mettre en évidence ci-après, la nature du ou des métaux, du ou des supports, du ou des promoteurs, et l'état de dispersion du métal sur le support conditionnent l'activité du catalyseur et donc les temps de contact.

Un certain nombre d'essais ont été réalisés avec différents types de catalyseurs. Les résultats de ces essais sont rapportés dans le tableau ci-après.

## TABLEAU

| Nature du catalyseur | 5% Ru/charbon actif | 10% Ru/SiO$_2$ | 5% Ru/Al$_2$O$_3$ | Co/Kieselguhr | 5%Pt/charbon actif | 5%Pt/SiO$_2$ | 5%Pt/SiO$_2$ |
|---|---|---|---|---|---|---|---|
| Masse du catalyseur, g | 0,6 | 0,5 | 0,6 | 0,6 | 0,6 | 0,1 | 0,1 |
| Température, °C | 200 | 130 | 150 | 250 | 275 | 250 | 250 |
| Durée d'exposition au CH$_4$, s | 480 | 300 | 480 | 480 | 480 | 300 | 30 |
| Durée d'exposition à H$_2$, s | 12 | 12 | 12 | 12 | 12 | 12 | 10 |
| Débit CH$_4$, l/h | 15,0 | 6,0 | 15,0 | 18,0 | 12,0 | 24,0 | 24,0 |
| Débit H$_2$, l/h | 7,2 | 7,2 | 7,2 | 7,2 | 7,2 | 7,2 | 7,2 |
| Quantité de CH$_4$ converti/g cata g | $1{,}28.10^{-3}$ | $0{,}11.10^{-3}$ | $1{,}34.10^{-3}$ | $0{,}58.10^{-3}$ | $0{,}95.10^{-3}$ | $0{,}24.10^{-3}$ | $2{,}4.10^{-3}$ |
| Conversion,% (CH$_4$ converti/CH$_4$ entrant) | 0,10 | 0,03 | 0,11 | 0,04 | 0,08 | 0,16 | 1,7 |

Comme cela ressort du tableau ci-dessus, les meilleures conversions et donc les meilleurs rendements - puisque la sélectivité en mélange d'alcanes supérieurs est de 100% - sont obtenus avec un catalyseur à base de platine sur un support de silice.

Il faut noter que l'on peut adapter le présent procédé à la production d'hydrocarbures plus lourds (jusqu'à $C_7$) en augmentant le temps de contact avec le catalyseur.

Bien entendu, l'invention n'est nullement limitée aux exemples donnés ci-dessus.

## Revendications

1. Procédé de conversion du méthane en hydrocarbures supérieurs, caractérisé en ce qu'il consiste à exposer un catalyseur constitué essentiellement d'un métal appartenant à la série des métaux de transition et d'un support de grande surface spécifique qui est constitué notamment d'oxyde d'un métal réfractaire ou de charbon actif, à un flux de méthane à une température comprise entre 100° et 300°C avec un temps de contact d'au moins une seconde, puis à un flux d'hydrogène à une température comprise entre 100 et 300°C avec un temps de contact d'au moins une seconde.

2. Procédé selon la revendication 1, caractérisé en ce que le métal est choisi dans le groupe consistant en Pt, Ru, Pd, Ir, Rh, Re, Co, Ni, Fe, W, leurs mélanges mécaniques et/ou leurs alliages, et leurs promoteurs à métaux notamment alcalins ou alcalino-terreux.

3. Procédé selon la revendication 1, caractérisé en ce que le support de grande surface spécifique est constitué d'oxyde de métal réfractaire choisi notamment dans le groupe consistant en silice, alumine, silice-alumine, oxyde de zinc, oxyde de magnésium, oxyde de chrome, oxyde de zirconium et oxyde de terres rares, ou de charbon actif.

4. Procédé selon la revendication 1, caractérisé en ce que la température est de préférence comprise entre 130°C et 250°C.

5. Procédé selon la revendication 1, caractérisé en ce que le temps de contact est de préférence compris entre 12 secondes et 480 secondes.

6. Procédé selon l'une des revendications précédentes, caractérisé en ce qu'il consiste de plus à récupérer le flux d'hydrogène en séparant l'hydrogène des produits obtenus.

7. Procédé selon l'une des revendications précédentes, caractérisé en ce qu'il consiste en une mise en oeuvre successive et non interrompue des étapes de la revendication 1.

## Patentansprüche

1. Verfahren zur Umwandlulng von Methan in höhere Kohlenwasserstoffe, dadurch gekennzeichnet, dass es darin besteht, einen Katalysator, der im wesentlichen aus einem zu der Übergangsmetallreihe gehörenden Metall und aus einem Träger mit grosser spezifischer Fläche, der insbesondere aus einem Oxid eines hitzebeständigen Metalls oder aus Aktivkohle besteht, einem Methanstrom bei einer zwischen 100° und 300°C liegenden Temperatur mit einer Berührungszeit von wenigstens einer Sekunde und sodann einem Wasserstoffstrom bei einer zwischen 100 und 300°C liegenden Temperatur mit einer Berührungsdauer von wenigstens einer Sekunde auszusetzen.

2. Verfahren nach Anspruch 1, dadurch gekennzeichnet, dass das Metall in der aus Pt, Ru, Pd, Ir, Rh, Re, Co, Ni, Fe, W, aus ihren mechanischen Gemischen und/oder ihren Legierungen und aus ihren Promotoren mit insbesondere Alkali- und Erdalkalimetalle bestehenden Gruppe gewählt wird.

3. Verfahren nach Anspruch 1, dadurch gekennzeichnet, dass der Träger mit grosser spezifischer Fläche aus einem hitzebeständigen Metalloxid besteht, das insbesondere in der aus Siliziumdioxid, Aluminiumdioxid, Siliziumdioxid-Aluminiumdioxid, Zinkoxid, Magnesiumoxid, Chromoxid, Zirkoniumoxid und Oxid von seltenen Erden, oder Aktivkohle bestehenden Gruppe gewählt wird.

4. Verfahren nach Anspruch 1, dadurch gekennzeichnet, dass die Temperatur vorzugsweise zwischen 130°C und 250°C liegt.

**5.** Verfahren nach Anspruch 1, dadurch gekennzeichnet, dass die Berührungsdauer vorzugsweise zwischen 12 Sekunden und 480 Sekunden liegt.

**6.** Verfahren nach einem der vorangehenden Ansprüche, dadurch gekennzeichnet, dass es ausserdem darin besteht, den Wasserstoffstrom zurückzugewinnen, indem der Wasserstoff von den erhaltenen Erzeugnissen getrennt wird.

**7.** Verfahren nach einem der vorangehenden Ansprüche, dadurch gekennzeichnet, dass es in einem aufeinanderfolgenden und ununterbrochenen Einsatz der Schritte des Anspruches 1 besteht.

**Claims**

**1.** Method of conversion of methane into higher hydrocarbons, characterized in that it consists in exposing a catalyst consisting essentially of a metal belonging to the series of the transition metals and of a support with a great specific surface area which consists in particular of oxide of a refractory metal or of activated charcoal, to a flow of methane at a temperature lying between 100° and 300°C with a contact time of at least one second and then to a flow of hydrogen at a temperature lying between 100 and 300°C with a contact time of at least one second.

**2.** Method according to claim 1, characterized in that the metal is selected from the group consisting of Pt, Ru, Pd, Ir, Rh, Re, Co, Ni, Fe, W, their mechanical mixtures and/or their alloys and their promoters with in particular alkaline or alkaline-earth metals.

**3.** Method according to claim 1, characterized in that the support with a great specific surface area consists of oxide of refractory metal selected in particular from the group consisting of silica, alumina, silica-alumina, zinc oxide, magnesium oxide, chromium oxide, zirconium oxide and oxide of rare earths or of activated charcoal.

**4.** Method according to claim 1, characterized in that the temperature is lying preferably between 130°C and 250°C.

**5.** Method according to claim 1, characterized in that the contact time is preferably lying between 12 seconds and 480 seconds.

**6.** Method according to one of the foregoing claims, characterized in that it consists in addition in recovering the flow of hydrogen by separating the hydrogen from the products obtained.

**7.** Method according to one of the foregoing claims, characterized in that it consists in a successive and non-interrupted use of the steps of claim 1.